# EUROPÄISCHE PATENTSCHRIFT

(11) **EP 2 097 048 B1**
(45) Veröffentlichungstag und Bekanntmachung des Hinweises auf die Patenterteilung: **01.09.2010**
(21) Anmeldenummer: 07872086.9
(22) Anmeldetag: 12.12.2007
(51) Int. Cl.: A61F 2/60, A61F 5/01

(54) **ORTHOPÄDIETECHNISCHES GERÄT**
ORTHOPEDIC DEVICE
APPAREIL ORTHOPÉDIQUE

(30) Priorität: 13.12.2006 DE 102006059206
(43) Veröffentlichungstag der Anmeldung: 09.09.2009
(73) Patentinhaber: Otto Bock HealthCare GmbH, 37115 Duderstadt (DE)
(72) Erfinder: ALBRECHT-LAATSCH, Erik, 37077 Göttingen (DE); CARSTENS, Ralf, 59557 Lippstadt (DE); NÖRTHEMANN,Jens, 37115 Duderstadt (DE); SCHILLING,Matthias, 37345 Weißenborn-Lüderode (DE)
(74) Vertreter: Stornebel, Kai
(86) Internationale Anmeldenummer: PCT/IB2007/004480
(87) Internationale Veröffentlichungsnummer: WO 2008/072095

(56) Entgegenhaltungen:
- EP-A- 1 532 951
- WO-A-2006/099580
- WO-A-2007/025116
- US-A1- 2006 249 315

## Beschreibung

Die Erfindung betrifft ein orthopädietechnisches Gerät, insbesondere Orthesen oder Prothesen, mit zueinander verschwenkbar gelagerten Komponenten.

Orthopädietechnische Geräte mit zwei gelenkig verbundenen und zueinander schwenkbar gelagerten Komponenten sind für bestimmte Anwendungsfälle mit Sensoren und Aktuatoren ausgestattet. Auf Grundlage der ermittelten Sensordaten können Veränderungen an dem orthopädietechnischen Gerät vorgenommen werden, beispielsweise können die Dämpfungseigenschaften eines Gelenkes in Abhängigkeit von den ermittelten Sensordaten verändert werden. Ebenfalls ist es möglich, bewegungsunterstützende Maßnahmen über Aktuatoren einzuleiten oder die orthopädietechnischen Geräte lediglich mit Sensoren auszustatten, deren Sensorsignale z.B. drahtlos auf einen Empfänger übertragen werden können. Dadurch wird eine Überwachung oder Überprüfung des Gerätes während der Benutzung für den jeweiligen Prothesennutzer möglich, so dass gegebenenfalls notwendige Anpassungen einfacher vorgenommen werden können.

In der Regel werden die Sensoren oder Aktuatoren bzw. Sendeeinrichtungen mit elektrischer Energie eines Akkumulators versorgt, der einen ungestörten Betrieb über einen festgelegten Zeitraum gewährleistet. Dieser Zeitraum umfasst den üblichen Aktivitätszeitraum eines Tages, während der Akkumulator während eines Passivzeitraumes aufgeladen wird. Die für den Betrieb der orthopädietechnischen Geräte verwendbaren Akkumulatoren sind sowohl in ihrer Baugröße als auch in ihrem Gewicht begrenzt, da in orthopädietechnischen Geräten möglichst kleine und leichte Komponenten verbaut werden sollen. Daraus ergibt sich eine begrenzte Ladekapazität des Akkumulators. Es ist daher problematisch, über eine Minimalausstattung hinaus zusätzliche Sensoren oder Aktuatoren vorzusehen oder eine wirksame Unterstützung einer Bewegung vorzusehen. Ebenfalls sind die Einsatzzeiten begrenzt.

Aus der US 5,703,474 ist es bekannt, eine mechanische Kraft in elektrische Energie zu wandeln und damit einen Akkumulator aufzuladen. Es ist auch bekannt, die beim Laufen durch das Gewicht eines Menschen entstehende Bodenreaktionskraft zur piezoelektrischen Energiegewinnung zu verwenden. In IEEE-MICRO, Heft Mai - Juni 2001, Seiten 30 - 42 ist eine piezoelektrische Anordnung beschrieben, die an einer biegsamen Folie an einer Schuhsohle angebracht ist. Die gewonnene Energie wird dazu verwendet, die Schuhsohle zu beleuchten. Die mit der Verwendung des piezoelektrischen Elements auf einer biegsamen Folie gewinnbare elektrische Energie ist minimal und daher für die Anwendung in einem orthopädietechnischen Gerät nicht geeignet.

Die Patentschrift US2006/0 249 315 offenbart des weiteren ein orthopädie technisches Gerät gemäß des Oberbegriffes von Anspruch 1.

Aufgabe der vorliegenden Erfindung ist es, ein orthopädietechnisches Gerät mit zwei zueinander verschwenkbar gelagerten Komponenten und diesen gegebenenfalls zugeordneten Sensoren oder Aktuatoren so zu verbessern, dass eine ausreichende Energieversorgung sichergestellt und eine möglichst leichte Konstruktion erzielt wird.

Diese Aufgabe wird durch ein orthopädietechnisches Gerät mit den Merkmalen des Anspruchs 1 gelöst. Vorteilhafte Ausgestaltungen und Weiterbildungen der Erfindung sind in den Unteransprüchen aufgeführt.

Das erfindungsgemäße orthopädietechnische Gerät mit zwei zueinander verschwenkbar gelagerten Komponenten sieht vor, dass daran ein Generator zur Erzeugung elektrischer Energie angeordnet ist. Der Generator wird über eine Übertragungseinrichtung angetrieben, die eine Relativbewegung der beiden Komponenten zueinander überträgt. Bei der Bewegung eines orthopädietechnischen Gerätes, insbesondere beim Gehen, werden innerhalb der Gelenke kurzfristig relativ hohe Energiebeträge in Gestalt von Biegemomenten bereitgestellt. Diese hohen Energiebeträge ergeben sich aus dem Impuls beim Auftreten und Abbremsen des Körpergewichtes und den Hebelverhältnisses an dem jeweiligen Gelenk. Ebenfalls bei anderen Prothesen- oder Ortheseneinrichtungen, beispielsweise an den oberen Extremitäten oder bei einer Lumbalorthese, können hohe Biegemomente auftreten. Um diese kurzzeitigen Energieeinstiege ausnutzen zu können, ist eine Übertragungseinrichtung vorgesehen, die eine translatorische oder rotatorische Bewegung so umsetzt, dass ein Generator zur Erzeugung elektrischer Energie bevorzugt drehend angetrieben wird. Dieser Antrieb erfolgt bevorzugt dergestalt, dass nur eine Drehrichtung für die Antriebswellen des Generators ermöglicht wird, um den Aufwand für eine Drehrichtungsumkehr des Generators zu minimieren. Ebenfalls kann durch einen Antrieb in nur einem Drehsinn ein Kraftspeicher, insbesondere eine Feder, leichter beaufschlagt insbesondere gespannt und wirksam eingesetzt werden. Der Generator ist bevorzugt als ein Wechselstromgenerator ausgebildet, der an der Orthese oder Prothese befestigt ist und die energieverbrauchenden Komponenten, beispielsweise Aktuatoren oder Sensoren mit Energie versorgt. Der Generator arbeitet dabei nach dem Dynamoprinzip der elektromagnetischen Induktion und kann alternativ als Gleichstromgenerator ausgebildet sein.

Eine weitere Vergleichmäßigung der Energiebereitstellung durch den Generator wird dadurch erreicht, dass dem Generator ein Kraftspeicher vorgeschaltet ist. Der Kraftspeicher ist bevorzugt als Feder, insbesondere als Spiral- oder Wendelfeder ausgebildet und dient zur Glättung bzw. zum Auffangen von Bewegungsimpulsen. Solche Federn sind aus dem Uhrenbau hinlänglich bekannt und gewährleisten, dass der Generator gegebenenfalls über das Getriebe mit einer entsprechend hohen Drehzahl betrieben wird, solange wie die Vorspannung der Feder ausreichend hoch ist. Da durch den Kraftspeicher sowohl das Getriebe als auch der Generator in Bewegung gehalten werden, treten keine Haftreibungseffekte oder Anfahrverluste auf, was den Wirkungsgrad weiter erhöht. Zur Erhöhung des Wirkungsgrads und zur Vergleichmäßigung des Antriebes des Generators ist diesem bevorzugt ein Getriebe vorgeschaltet, das die relativ kurze Bewegungslänge zweier zueinander verschwenkbarer Komponenten in eine an den Generator angepasste Drehzahl übersetzt.

Eine Weiterbildung der Erfindung sieht vor, dass dem Generator eine Sperreinrichtung zugeordnet ist, die einen Antrieb des Generators sperrt, wenn der Kraftspeicher ein zu geringes Kraftniveau aufweist. Dadurch wird gewährleistet, dass beispielsweise bei einem Federkraftspeicher erst ab einer Mindestspannung der Feder der Generator in Betrieb gesetzt wird. Dies kann durch eine mechanische Sperre des Generators, des Kraftspeichers oder eines zwischengeschalteten Getriebes oder durch eine elektrische Sperrschaltung, die den sperrt, beispielsweise eine Kurzschlussschaltung, erfolgen

Um zu gewährleisten, dass der Generator oder Kraftspeicher stets in einer gleichbleibenden Drehrichtung angetrieben wird, weist die Übertragungseinrichtung zumindest einen Freilauf auf, der die Kraftübertragung in einer festgelegten Drehrichtung sperrt und nur eine Kraftübertragung in der anderen, freigegebenen Drehrichtung zulässt. Bei alternierenden Bewegungen, wie sie bei Gelenkeinrichtungen im Rahmen der Flexion und Extension auftreten, wird auf diese Art und Weise ein Antrieb in einer Rotationsrichtung gewährleistet.

In einer Ausgestaltung der Erfindung ist es vorgesehen, dass die Übertragungseinrichtung eine Schubstange aufweist, die eine Drehung zweier Komponenten, beispielsweise eines Fußteiles und eines Unterschenkelteiles oder eines Oberteiles und eines Unterteiles einer Kniegelenkseinrichtung in eine Schubbewegung umsetzt. Diese Schubbewegung oder translatorische Bewegung erfolgt alternierend, wobei durch eine entsprechende Steuerung der Schubstange gewährleistet wird, dass ein Antrieb des Generators oder ein Beaufschlagen einer Kraftspeichereinrichtung nur in einer Richtung erfolgt. Durch die Schubstange kann eine Übersetzung verwirklicht werden, so dass die relativ kleinen Verdrehwinkel in große translatorische Bewegungen übersetzt werden können. Ebenfalls ist es möglich, dass die Schubstange über ein Gelenkgetriebe mit dem Generator bzw. dem vorgeschalteten Getriebe verbunden ist. Um eine Beaufschlagung des Generators bzw. des Kraftspeichers oder des Getriebes mit einer Verstellkraft durch die Schubstange nur in einer Richtung zu gewährleisten, kann der Schubstange ein Ratschenmechanismus zugeordnet sein, der nur in einer Richtung wirksam ist, in der anderen jedoch durchrutscht. Statt einer Umwandlung durch eine Schubstange kann die Übertragungseinrichtung die Drehbewegung direkt auf den Generator oder einen vorgeschalteten Kraftspeicher oder ein vorgeschaltetes Getriebe übertragen. Die Übertragung der Verschwenkung zweier Komponenten kann über unterschiedliche Mittel erfolgen, beispielsweise Zahn- oder Reibräder oder Zugmitteltriebe wie Ketten, Bänder oder Zahnriemen. Ebenfalls können die verschiedenen Übertragungsmittel miteinander kombiniert werden, um geeignete Übersetzungen, Verdrehwinkel oder Verschiebewege zu verwirklichen oder die Kräfte und Momente an die gewünschten Stellen zu leiten.

Weiterhin ist es möglich, dass die Übertragungseinrichtung eine Doppelzahnradanordnung mit zumindest einer Schalteinrichtung aufweist, die eine Kraftübertragung auf eine Abtriebswelle nur in eine Drehrichtung herstellt oder ermöglicht, so dass die Zahnräder in einer Kraftrichtung wirksam sind. Dabei ist vorgesehen, dass bei einem Zahnrad eine Drehrichtungsumkehr stattfindet, am einfachsten durch ein zwischengeschaltetes Zahnrad, um eine einheitliche Drehrichtung der Abtriebswelle und damit des Generators sicherzustellen. Durch eine entsprechende Umschaltung oder beispielsweise Kulissenführung ist es möglich, dass translatorische oder rotatorische Bewegungen in unterschiedlichen Richtungen auf je ein Zahnrad übertragen werden, die durch geeignete Übersetzungseinrichtungen die Antriebswelle nur in eine Drehrichtung drehen und in der anderen Drehrichtung gesperrt oder nicht angetrieben werden. Statt einer Schaltung der Zahnräder können diese auch mit einem Freilauf versehen sein und gleichzeitig angetrieben werden, wobei ein Zahnrad mit einer Einrichtung zur Drehrichtungsumkehr versehen ist, so dass bei alternierenden Bewegungen ein Antrieb nur in einer Richtung stattfindet.

Eine besonders einfache Art und Weise einer Kraftausnutzung und Kraftübertragung in nur eine Drehrichtung liegt vor, wenn ein Kegelradgetriebe vorgesehen ist, bei dem ein Antriebskegelrad mit zwei Abtriebskegelrädern gekoppelt ist. Die Abtriebskegelräder sind auf einer gemeinsamen Abtriebswelle gelagert und mit je einem Freilauf versehen, der so ausgestaltet ist, dass die Antriebswelle in nur einer Drehrichtung mit einem Drehmoment beaufschlagt wird. Da beide Abtriebsräder bei alternierenden Drehbewegungen alternierend angetrieben werden, wird bei der Drehung in einer Drehrichtung nur das eine und bei der Drehung in die andere Drehrichtung das gegenüberliegende Abtriebszahnrad kraftübertragend mit der Abtriebswelle gekoppelt.

Der Generator kann mit einem Akkumulator oder mit einem Kondensator gekoppelt sein, um neben einer mechanischen Kraftspeicherung auch eine elektrische Kraftspeicherung vorzusehen. Bei einem Wechselstromgenerator ist ein Gleichrichter vorzusehen, um einen Akkumulator aufzuladen. Insbesondere bei einer zwischengeschalteten Feder kann ein konstanter Abtrieb über eine zusätzliche mechanische Bremse kontrolliert werden. Ebenfalls ist es möglich, über eine Kurzschlussschaltung im Generator den Abtrieb zu steuern. Durch den konstanten Antrieb des Generators ist eine im Wesentlichen gleichbleibende Energieerzeugung möglich, so dass auf einen Transformator verzichtet werden kann, was sich positiv sowohl auf die Baugröße als auch auf das Gewicht auswirkt.

Nachfolgend werden Ausführungsbeispiele der Erfindung anhand der Figuren näher erläutert. Es zeigen:
- Figuren 1a -1c-: drei Ansichten einer ersten Ausführungsform mit direkter Drehbewegungsübertragung;
- Figuren 2a - 2c -: eine Variante mit einer translatorischen Übertragung;
- Figuren 3a und 3b -: Ansichten einer Anordnung an einem Prothesenfuß;
- Figuren 4a- und 4b -: eine Variante der Figur 3;
- Figur 5 -: eine schematische Darstellung einer Übertragungseinrichtung mit Schubstange; sowie
- Figuren 6a - 6c -: verschiedene Ansichten einer Variante der Übertragungseinrichtung.

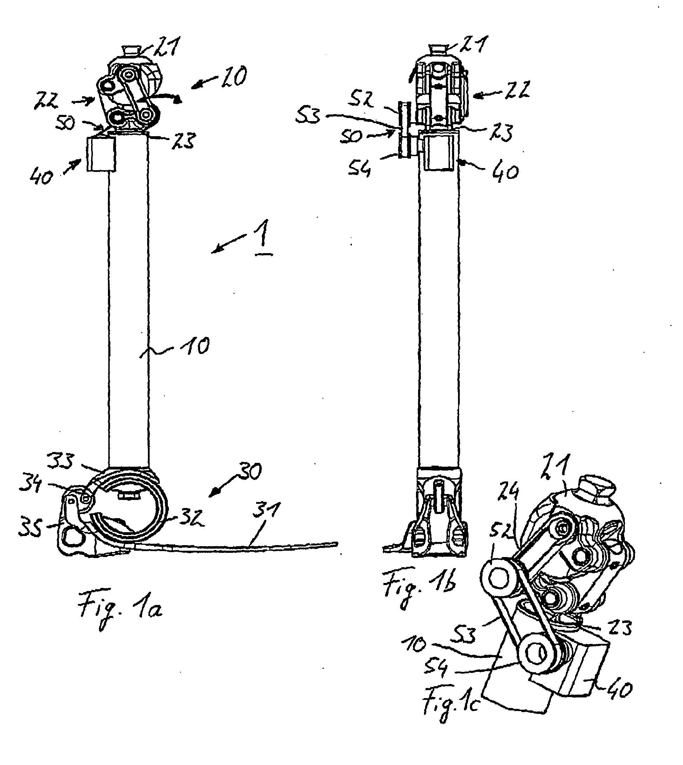

Die Figur 1a zeigt in Seitenansicht ein orthopädietechnisches Gerät 1 in Gestalt einer Beinteilprothese. An einem Unterschenkelschaft 10 ist an den oberen Ende ein Prothesenkniegelenk 20 und an dem unteren Ende ein Prothesenfuß 30 angeordnet. Ein Sohlenbereich 31 ist über eine Feder 32 an einem Anschlusselement 33 festgelegt. Über eine Gelenkeinrichtung 34 ist der Unterschenkelschaft 10 gelenkig an einem Fersenkörper 35 gelagert, an dem auch das Sohlenteil 31 befestigt ist.

An dem oberen Ende des Unterschenkelschaftes 10 ist das Prothesenkniegelenk 20 mit oberen Anschlussmitteln 21 zur Aufnahme eines Oberschenkelschaftes oder dergleichen angeordnet. Untere Anschlussmittel 23 legen das Prothesenkniegelenk 20 an dem Unterschenkelschaft 10 fest. Ein Mehrgelenksystem 22 sorgt für eine gelenkige Verbindung des oberen Anschlussmittels 21 zu dem unteren Anschlussmittel 23 bzw. dem Unterschenkelschaft 10.

An dem Unterschenkelschaft 10 ist eine Generatoreinheit 40 befestigt, die über eine Übertragungseinrichtung 50 angetrieben wird. In der Figur 1b, in der in rückwärtiger Draufsicht das orthopädietechnische Gerät 1 dargestellt ist, ist zu erkennen, dass die Übertragungseinrichtung 50 seitlich neben dem Prothesenkniegelenk 20 angeordnet ist und zwei Räder 52, 54 aufweist, die über einen Zahnriemen 53 oder über einen anderen Zugmitteltrieb miteinander verbunden sind. Dies ist detaillierter in der Figur 1c zu erkennen. Das Prothesenkniegelenk 20 weist in dem Mehrgelenksystem 22 einen vorderen Lenker 24 auf, der relativ zu dem unteren Befestigungsmittel 23 und damit zu dem Unterschenkel 10 verschwenkbar gelagert ist. Das erste Antriebsrad 52 ist dabei drehfest an dem vorderen Lenker 24 oder seiner Drehachse befestigt, so dass bei einer Beugung oder Streckung des Prothesenkniegelenkes 20 eine Verdrehung des vorderen Lenkers 24 stattfindet. Die alternierenden Verschwenkbewegungen sind durch den Doppelpfeil in der Figur 1a angedeutet. Verschwenkt der vordere Lenker 24, ausgehend von der dargestellten gestreckten Stellung im Laufe einer Flexion des Prothesenkniegelenkes 20, in Uhrzeigerrichtung, dreht sich das Antriebsrad 52 korrespondierend und treibt den Zahnriemen 53 oder ein anderes Kraftübertragungselement entsprechend an. Ein Antriebsrad 54, das mit der Generatoreinheit 40 verbunden ist, wird korrespondierend alternativ um dessen Drehachse bewegt. Die Verdrehung des Antriebsrades 54 wird auf einen Generator übertragen, was später näher erläutert wird. Gegebenenfalls kann die Bewegungsenergie über einen Kraftspeicher in Gestalt einer Feder gespeichert werden. Ebenfalls ist es möglich, eine Getriebeeinheit zwischenzuschalten. Die Drehbewegung des vorderen Lenkers 24 wird über den Zugmitteltrieb 53 in eine unmittelbare Drehbewegung des Antriebsrades 54 und damit einer Antriebswelle für den Generator umgewandelt.

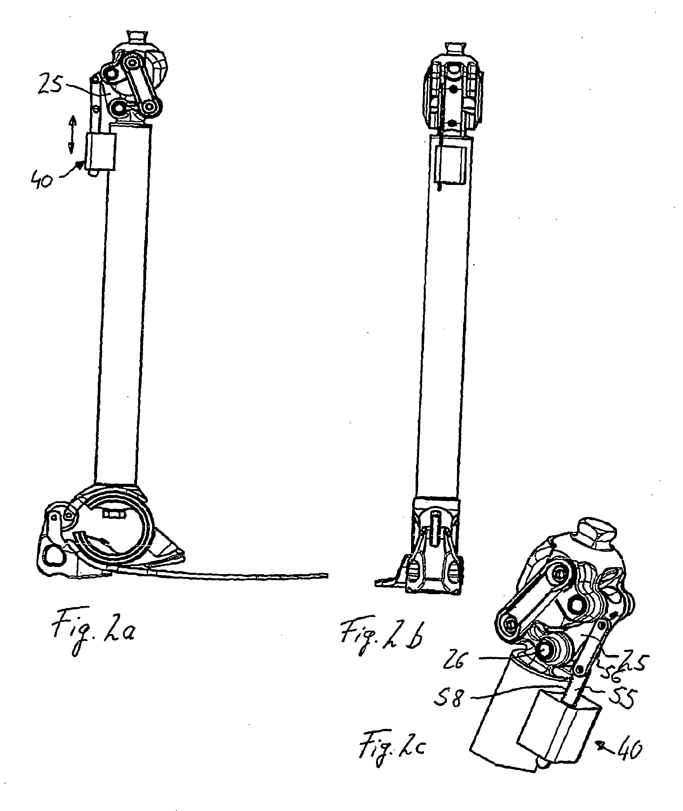

Eine Variante der Erfindung ist in den Figuren 2a bis 2c dargestellt, die entsprechend den Figuren 1a bis 1c aufgebaut sind. Statt einer Übertragung einer Verschwenkung eines vorderen Lenkers 24 wird eine Relativverschenkung eines hinteren Lenkers 25 um eine untere Drehachse 26 über zwei Schubstangen 55, 56 auf die Generatoreinheit 40 übertragen. Statt einer seitlichen Anordnung, wie sie in den Figuren 1a bis 1c dargestellt ist, wird das Kniegelenk 20 durch die in den Figuren 2a bis 2c dargestellte Anordnung in Sagittalebene hintereinander schmaler. An der unmittelbar in die Generatoreinheit 40 eingreifenden Schubstange 55 ist eine Verzahnung 58 ausgebildet, über die ein Generator mittelbar oder unmittelbar angetrieben wird.

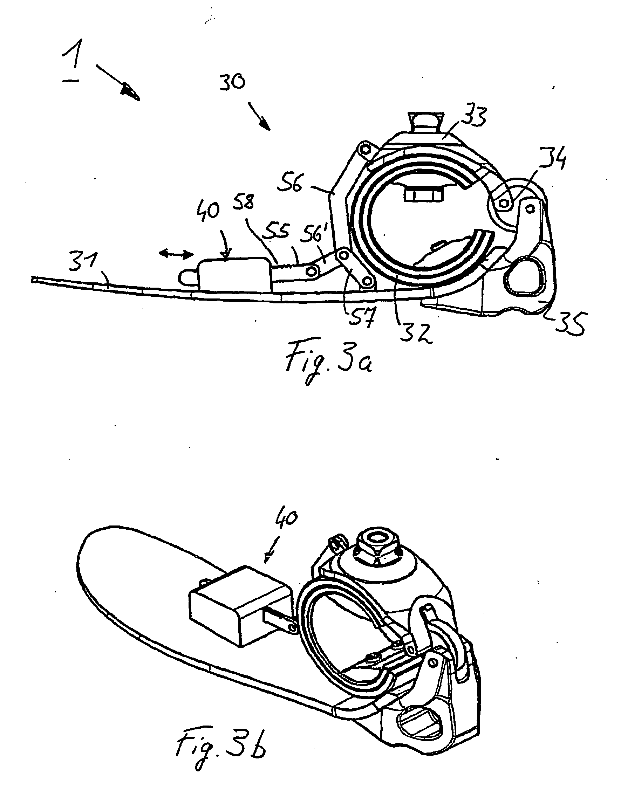

In den Figuren 3a und 3b ist ein Prothesenfuß 30 als orthopädietechnisches Gerät 1 gezeigt, bei dem die Energieumwandlung in elektrische Energie unmittelbar an dem Prothesenfuß 30 stattfindet. Die Generatoreinheit 40 ist auf dem Sohlenteil 31 angeordnet und über eine Zahnstange 55 mit einer Verzahnung 58 über ein Mehrtenkergetriebe 56, 56', 57 mit dem schwenkbar gelagerten oberen Anschlussteil 33 gekoppelt. Bei einer Belastung, beispielsweise Vorfußbelastung durch den nicht dargestellten Unterschenkelschaft 10, wird die Feder 32 komprimiert bzw. eingerollt, so dass das obere Anschlussmittel 33 um die Schwenkachse 34 relativ zu dem Fersenteil 35 und dem Sohlenteil 31 verschwenken kann. Dadurch wird über das Mehrlenkergetriebe 56, 56', 57 die Zahnstange 55 nach vorn bewegt. Verschwenkt das obere Anschlussmittel 33 zurück in Uhrzeigerrichtung in die dargestellte Ausgangsstellung, wird die Zahnstange 55 nach rechts gezogen. Die alternierende translatorische Bewegung der Zahnstange 55 ist durch den Doppelpfeil angedeutet. Innerhalb der Generatoreinheit 40 wird dann die translatorische Bewegung in eine rotatorische Bewegung umgesetzt und ein nicht dargestellter Generator angetrieben.

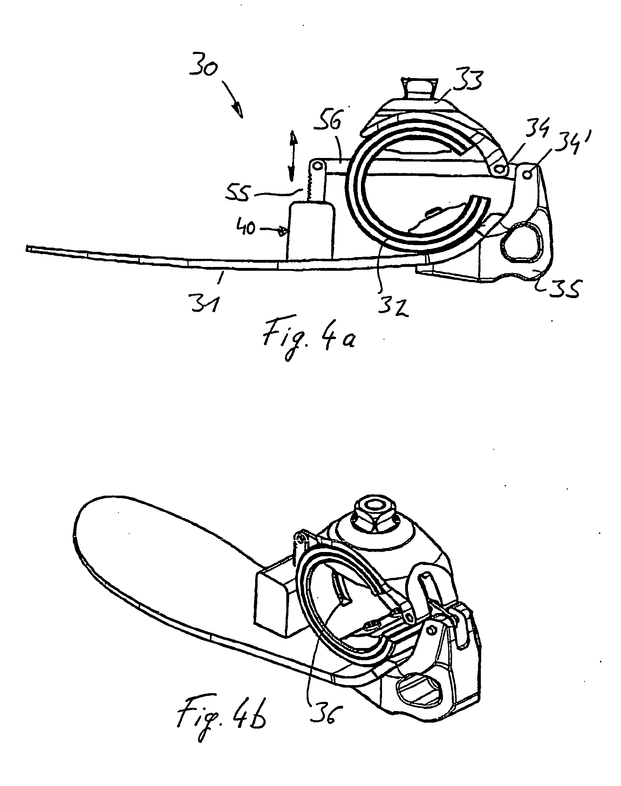

Eine alternative Ausgestaltung eines Prothesenfußes 30 ist in den Figuren 4a und 4b gezeigt, bei der das Oberteil 33 schwenkbar um eine Schenkachse 34 an einem Lagerbalken 56 gelagert ist, der wiederum gelenkig an einer vertikal orientierten Zahnstange befestigt ist. Der Lagerbalken 56 verschwenkt um die Schwenkachse 34' bei einer Belastung und bewirkt somit eine Bewegung der Zahnstange 55 in Richtung auf das Sohlenteil 31. Fällt die Belastung weg, wird durch die Feder 32 die dargestellte Ausgangsstellung wieder eingenommen, so dass die Zahnstange 55 nach oben verlagert wird. Dies ist durch den Doppelpfeil angedeutet. Der Tragbalken 56 ist dabei durch einen Schlitz 36 innerhalb der Feder 32 hindurchgeführt, wodurch sich ein sehr kompakter Aufbau realisieren lässt.

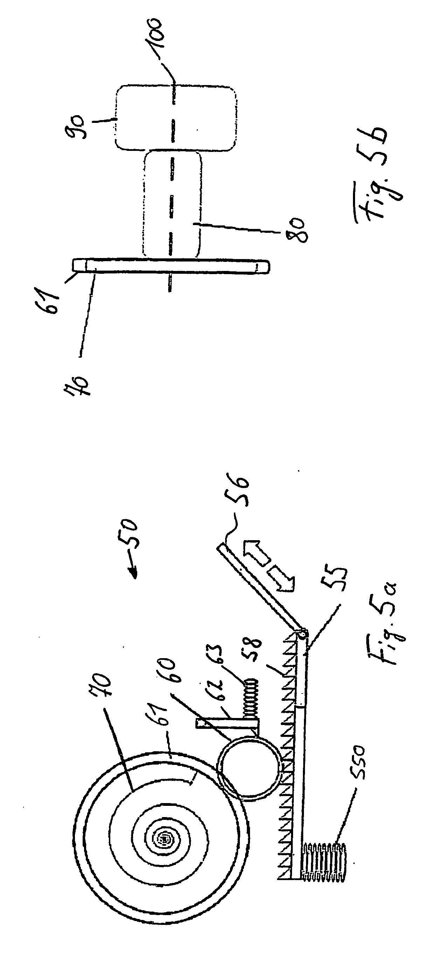

Die Figur 5 zeigt eine Prinzipdarstellung einer Übertragungseinrichtung 50 mit einer Schubstange 56, die eine alternierende, translatorische Bewegung ausführt. Die Schubstange 56 ist mit einer Zahnstange 55 gekoppelt, an der eine Verzahnung 58 in Gestalt einer Sägezahnverzahnung angeordnet ist. Die Zahnstange 55 kämmt ein Zahnrad 60, das wiederum in Eingriff mit einem weiteren Zahnrad 61 steht. Über diese zweite Zahnrad 61 wird eine Spiralfeder 70 als Kraftspeicher gespannt. Wird die Zahnstange 55 nach links verschoben, wird das Zahnrad 60 im Uhrzeigersinn verdreht, wodurch das zweite Zahnrad 61 entgegen dem Uhrzeigersinn gedreht wird und die Spiralfeder 70 spannt. Bei einer Bewegung nach rechts gleiten die Zähne 58 über das Zahnrad 60 hinweg, ohne eine Verstellung zu bewirken. Eine Rückstellfeder 550 presst die Zahnstange 55 dann wieder gegen das Zahnrad 60, um die Verzahnung 58 in Eingriff zu halten. Zur Absicherung ist eine mechanische Bremse 62 mit einer Feder 63 gegen das Zahnrad 60 vorgespannt, so dass eine Bewegung des Zahnrades 60 entgegen dem Uhrzeigersinn verhindert wird. Die Zahnräder 60, 61 können in unterschiedlichen Übersetzungen, gegebenenfalls durch ein dazwischengeschaltetes Getriebe miteinander gekoppelt sein.

In der Figur 5b ist in Seitenansicht die Zuordnung der Spiralfeder 70 über das Zahnrad 61 auf einer Drehachse 100 gezeigt. Die Drehfeder 70 treibt über ein Getriebe 80 mit einer hohen Untersetzung einen Gleichstromgenerator 90 an. Sobald die Getriebehaftreibung überwunden ist, kommt der Generator 90 durch die starke Untersetzung des Getriebes 80 in Rotation und erreicht bei entsprechender Vorspannung der Feder 70 eine hohe Drehzahl, die solange erhalten bleibt, wie die Spannung der Feder 70 ausreicht. Beim Gehen können so die im Prothesenfuß 30 oder in dem Prothesenkniegelenk 20 erreichten und entstehenden kurzzeitigen Energieanstiege durch die Feder 70 in Gestalt einer wiederholten Verdrehung der Feder 70 in eine gespannte Stellung gespeichert und langsam über das Getriebe 80 an den Generator 90 abgeben werden. Dies dient zu einer Glättung und zu einem Auffangen von Impulsen und zu einer Erhöhung des Gesamtwirkungsgrades.

Durch eine Bewegungssteuerung, beispielsweise durch eine Kulissenführung der Zahnstange 55, kann auch auf eine Ratschenausgestaltung des Bewegungsumsetzers in der Übertragungseinrichtung 50 verzichtet werden. Ebenfalls können alternierende Drehbewegungen über entsprechende Freilaufanordnungen und eine Umlenkung so umgewandelt werden, dass eine Antriebswelle für den Generator 90 bzw. für die Vorspannung der Feder 70 stets nur in einer Richtung gedreht wird.

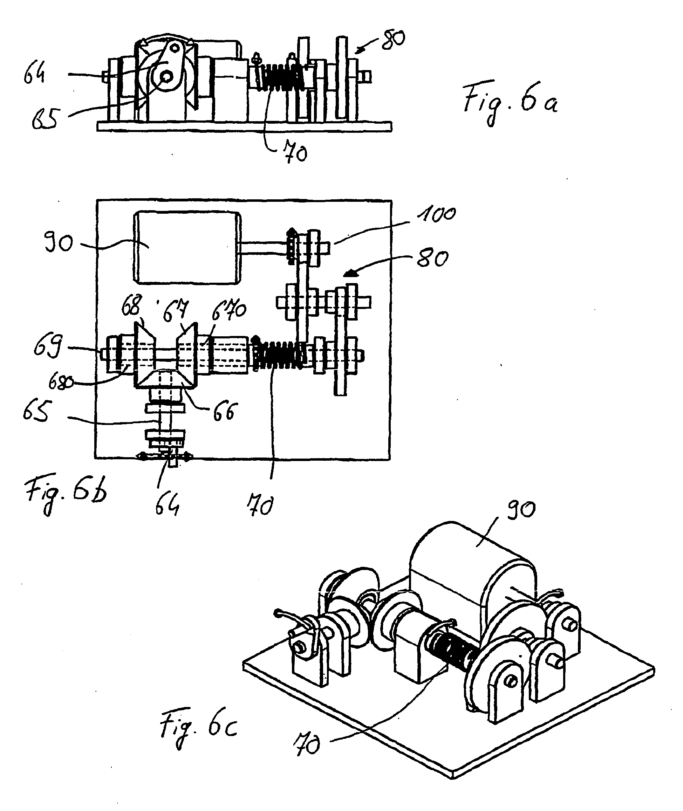

In den Figuren 6a bis 6c ist eine alternative oder ergänzende Umsetzung einer alternierenden translatorischen Bewegung in eine Drehbewegung dargestellt. Über eine Kurbel 64 wird eine Verschwenkbewegung in eine alternierende Drehbewegung auf eine Welle 65 übertragen. Auf dieser Welle 65 ist ein Antriebskegelrad 66 gelagert, das korrespondierend zu der Drehbewegung der Welle 65 alternierende Drehbewegungen ausübt. In das Antriebsrad 66 greifen zwei auf einer gemeinsamen Abtriebswelle 69 gelagerte Abtriebsräder 67, 68 in Gestalt von Kegelzahnrädern ein, die jeweils mit Freilaufeinrichtungen 670, 680 ausgestattet sind. Dreht die Welle 65 und das Antriebszahnrad 66 in Uhrzeigerrichtung, gesehen in Blickrichtung der Figur 6a, bleibt das rechte Abtriebszahnrad 67 ohne Kraftübertragung, während das linke Abtriebszahnrad 68 die Abtriebswelle 69 entsprechend antreibt. Bei einer Schwenkbewegung der Kurbel 64 nach links, dreht die Welle 65 entgegen dem Uhrzeigersinn, wodurch das linke Zahnrad 68 über den Freilauf 680 aus einem kraftübertragenden Eingriff herausgenommen und stattdessen das rechte Zahnrad 67 kraftübertragend geschaltet wird. Dadurch wird sowohl bei einer Extension als auch bei einer Flexion der Komponenten des orthopädietechnischen Gerätes bei einer entsprechenden Kraftübertragung durch die Übertragungseinrichtung 50 die Abtriebswelle 69 in nur einer Drehrichtung verdreht.

Die Abtriebswelle 69 ist mit einer Spiral- oder Wendelfeder 70 als mechanischem Kraftspeicher gekoppelt, die wiederum mit einer Getriebeeinheit 80 gekoppelt ist. Über verschiedene Übersetzungsstufen der Getriebeeinheit 80 wird der Generator 90 angetrieben, wodurch die Bewegungsenergie durch die Flexion und Extension bzw. die Relativverlagerung der Komponenten des orthopädietechnischen Gerätes in elektrische Energie umgewandelt wird. Diese elektrische Energie kann unmittelbar an nicht dargestellte Verbraucher, beispielsweise Aktuatoren oder Sensoren oder Sendeeinrichtungen, übermittelt werden. Ebenfalls ist es möglich, dass die erzeugte elektrische Energie in Akkumulatoren oder Kondensatoren gespeichert werden.

Um einen konstanten Antrieb zu ermöglichen, kann die Feder 70 über eine mechanische Bremse kontrolliert werden. Über eine Kurzschlussschaltung innerhalb des Generators 90 kann der Antrieb ebenfalls gesteuert werden.

Statt der Umwandlung einer translatorischen Bewegung über eine Kurbel 64 kann die Antriebswelle 65 auch unmittelbar über einen Zahnriemen oder dergleichen rotatorisch angetrieben werden. Grundsätzlich ist es auch möglich, dass die Antriebswelle 65 oder gegebenenfalls auch die Generatorwelle 100 unmittelbar an einer Drehachse der verlagerten Komponente angeordnet ist.

## Patentansprüche

1. Orthopädietechnisches Gerät (1) mit zwei zueinander verschwenkbar gelagerten Komponenten (24, 10; 25, 10; 33, 35), wobei an dem orthopädietechnischen Gerät (1) ein Generator (90) zur Erzeugung elektrischer Energie angeordnet ist, der über eine Übertragungseinrichtung (50), die eine Relativbewegung der beiden Komponenten (24, 10; 25, 10; 33, 35) zueinander überträgt, angetrieben wird, wobei dem Generator (90) ein Kraftspeicher (70) vorgeschaltet ist, **dadurch gekennzeichnet, dass** die Übertragungseinrichtung (50) eine Kraftübertragung nur in einer Richtung zulässt.

2. Orthopädietechnisches Gerät nach Anspruch 1, **dadurch gekennzeichnet, dass** es als eine Prothese oder Orthese ausgebildet ist.

3. Orthopädietechnisches Gerät nach Anspruch 1 oder 2, **dadurch gekennzeichnet, dass** der Generator (90) als Gleich- oder Wechselstromgenerator ausgebildet ist.

4. Orthopädietechnisches Gerät nach einem der voranstehenden Ansprüche, **dadurch gekennzeichnet, dass** dem Generator (90) ein Getriebe (80) vorgeschaltet ist.

5. Orthopädietechnisches Gerät nach einem der voranstehenden Ansprüche, **dadurch gekennzeichnet, dass** eine Sperreinrichtung für den Generator (90) vorgesehen ist, die eine Erzeugung elektrischer Energie sperrt, bis der Kraftspeicher (70) ein Kraftmindestniveau erreicht hat.

6. Orthopädietechnisches Gerät nach einem der voranstehenden Ansprüche, **dadurch gekennzeichnet, dass** der Kraftspeicher (70) als eine Feder, insbesondere als eine Spiralfeder ausgebildet ist.

7. Orthopädietechnisches Gerät nach einem der voranstehenden Ansprüche, **dadurch gekennzeichnet, dass** die Übertragungseinrichtung (50) zumindest einen Freilauf (670, 680) aufweist, der eine Kraftübertragungsrichtung sperrt

8. Orthopädietechnisches Gerät nach einem der voranstehenden Ansprüche, **dadurch gekennzeichnet, dass** die Übertragungseinrichtung (50) eine Schubstange (55) aufweist, die die relative Drehbewegung der beiden Komponenten (24, 10; 25, 10; 33, 35) zueinander in eine translatorische Bewegung überträgt.

9. Orthopädietechnisches Gerät nach Anspruch 8, **dadurch gekennzeichnet, dass** die Schubstange (55) über einen Ratschenmechanismus mit dem Generator (90) gekoppelt ist.

10. Orthopädietechnisches Gerät nach einem der Ansprüche 1 bis 8, **dadurch gekennzeichnet, dass** die Übertragungseinrichtung (50) eine Doppelzahnradanordnung (67, 68) mit zumindest einer Schalteinrichtung aufweist, die eine Kraftübertragung auf eine Abtriebswelle (69) nur in einer Drehrichtung herstellt.

11. Orthopädietechnisches Gerät nach Anspruch 10, **dadurch gekennzeichnet, dass** das Doppelzahnradgetriebe (66, 67, 68) als ein Kegelradgetriebe ausgebildet ist, das ein alternierend angetriebenes Antriebskegelrad (66) und zwei Abtriebskegelräder (67, 68) aufweist, die über jeweils einen Freilauf (670, 680) auf einer Abtriebswelle (69) gelagert sind.

12. Orthopädietechnisches Gerät nach einem der voranstehenden Ansprüche, **dadurch gekennzeichnet, dass** der Generator (90) mit einem Akkumulator oder Kondensator gekoppelt ist.

## Claims

1. Orthopedic device (1) with two components (24, 10; 25, 10; 33, 35) which are mounted so as to be pivotable relative to each other, a generator (90) for generating electric power being arranged on the orthopedic device (1) and being driven via a transmission mechanism (50), which transmits a relative movement of the two components (24, 10; 25, 10; 33, 35) to each other, and an energy accumulator (70) being mounted upstream of the generator (90), **characterized in that** the transmission mechanism (50) allows energy to be transmitted only in one direction.

2. Orthopedic device according to Claim 1, **characterized in that** it is designed as a prosthesis or orthosis.

3. Orthopedic device according to Claim 1 or 2, **characterized in that** the generator (90) is designed as a direct current or alternating current generator.

4. Orthopedic device according to one of the preceding claims, **characterized in that** a gear arrangement (80) is mounted upstream of the generator (90).

5. Orthopedic device according to one of the preceding claims, **characterized in that** a blocking mechanism for the generator (90) is provided which blocks the generation of electric power until the energy accumulator (70) has reached a minimum energy level.

6. Orthopedic device according to one of the preceding claims, **characterized in that** the energy accumulator (70) is designed as a spring, in particular as a helical spring.

7. Orthopedic device according to one of the preceding claims, **characterized in that** the transmission mechanism (50) has at least one free-wheel (670, 680) that blocks one direction of energy transmission.

8. Orthopedic device according to one of the preceding claims, **characterized in that** the transmission mechanism (50) has a connecting rod (55), which converts the relative rotation movement of the two components (24, 10 ; 25, 10 ; 33, 35) to each other into a translation movement.

9. Orthopedic device according to Claim 8, **characterized in that** the connecting rod (55) is coupled to the generator (90) via a ratchet mechanism.

10. Orthopedic device according to one of Claims 1 to 8, **characterized in that** the transmission mechanism (50) has a double gearwheel arrangement (67, 68) with at least one switch mechanism, which produces energy transmission to an output shaft (69) only in one direction of rotation.

11. Orthopedic device according to Claim 10, **characterized in that** the double gearwheel arrangement (66, 67, 68) is designed as a bevel gear arrangement, which has an alternately driven bevel drive wheel (66) and two bevel output wheels (67, 68), which are mounted on an output shaft (69) in each case via a free-wheel (670, 680).

12. Orthopedic device according to one of the preceding claims, **characterized in that** the generator (90) is coupled to an accumulator or capacitor.

## Revendications

1. Appareil orthopédique (1) comprenant deux composants (24, 10 ; 25, 10 ; 33, 35) montés pivotables l'un par rapport à l'autre, un générateur (90) étant monté sur l'appareil orthopédique (1) pour produire de l'énergie électrique, générateur qui est entraîné par l'intermédiaire d'un dispositif de transmission (50) qui transmet un mouvement relatif des deux composants (24, 10 ; 25, 10 ; 33, 35) l'un par rapport à l'autre, un accumulateur d'énergie (70) étant placé en amont du générateur (90), **caractérisé en ce que** le dispositif de transmission (50) ne permet une transmission de force que dans une direction.

2. Appareil orthopédique selon la revendication 1, **caractérisé en ce qu'**il est réalisé en tant que prothèse ou orthèse.

3. Appareil orthopédique selon la revendication 1 ou 2, **caractérisé en ce que** le générateur (90) est réalisé en tant que générateur de courant alternatif ou continu.

4. Appareil orthopédique selon l'une des revendications précédentes, **caractérisé en ce qu'**une transmission (80) est montée en amont du générateur (90).

5. Appareil orthopédique selon l'une des revendications précédentes, **caractérisé en ce qu'**il est prévu pour le générateur (90) un dispositif de blocage qui bloque une production d'énergie électrique jusqu'à ce que l'accumulateur d'énergie (70) ait atteint un niveau minimum d'énergie.

6. Appareil orthopédique selon l'une des revendications précédentes, **caractérisé en ce que** l'accumulateur d'énergie (70) est réalisé en tant que ressort, en particulier un ressort en spirale.

7. Appareil orthopédique selon l'une des revendications précédentes, **caractérisé en ce que** le dispositif de transmission (50) présente au moins une roue libre (670, 680) qui bloque une direction de transmission de force

8. Appareil orthopédique selon l'une des revendications précédentes, **caractérisé en ce que** le dispositif de transmission (50) présente une bielle (55) qui transmet dans un mouvement de translation le mouvement rotatif relatif des deux composants (24, 10 ; 25, 10 ; 33, 35) l'un par rapport à l'autre.

9. Appareil orthopédique selon la revendication 8, **caractérisé en ce que** la bielle (55) est couplée au générateur (90) par l'intermédiaire d'un mécanisme à cliquet.

10. Appareil orthopédique selon l'une des revendications 1 à 8, **caractérisé en ce que** le dispositif de transmission (50) présente un agencement à deux roues d'engrenage (67, 68) avec au moins un dispositif de commutation, agencement qui établit seulement dans un sens de rotation une transmission de force à un arbre mené (69).

11. Appareil orthopédique selon la revendication 10, **caractérisé en ce que** la transmission à deux roues d'engrenage (66, 67, 68) est formée par une transmission à pignon conique qui présente un pignon conique d'entraînement (66) entraîné alternativement, et deux pignons coniques menés (67, 68) qui sont montés sur un arbre mené (69) chacun par l'intermédiaire d'une roue libre (670, 680).

12. Appareil orthopédique selon l'une des revendications précédentes, **caractérisé en ce que** le générateur (90) est couplé avec un accumulateur ou un condensateur.
